# EUROPÄISCHE PATENTSCHRIFT

(11) **EP 0 554 255 B1**
(45) Veröffentlichungstag und Bekanntmachung des Hinweises auf die Patenterteilung: **17.01.1996**
(21) Anmeldenummer: 91913000.5
(22) Anmeldetag: 27.07.1991
(51) Int. Cl.: C12M 3/00, C12M 1/12

(54) **VORRICHTUNG ZUR BEAUFSCHLAGUNG FLÜSSIGER MEDIEN MIT GASFÖRMIGEN STOFFEN**
DEVICE FOR FEEDING GASEOUS SUBSTANCES INTO LIQUIDS
DISPOSITIF POUR L'INTRODUCTION DE SUBSTANCES GAZEUSES DANS DES MILIEUX LIQUIDES

(30) Priorität: 28.08.1990 DE 4027126
(43) Veröffentlichungstag der Anmeldung: 11.08.1993
(73) Patentinhaber: BILLER, Edmund, D-83064 Raubling (DE); BOCK, Eberhard, D-22559 Hamburg (DE)
(72) Erfinder: BILLER, Edmund, D-83064 Raubling (DE); BOCK, Eberhard, D-22559 Hamburg (DE)
(74) Vertreter: Schupfner, Gerhard D.
(86) Internationale Anmeldenummer: DE9100608
(87) Internationale Veröffentlichungsnummer: WO9203534

(56) Entgegenhaltungen:
- EP-A- 232 975
- WO-A-89/12385
- WO-A-90/10690
- US-A- 3 184 395
- US-A- 3 941 662
- US-A- 4 416 993

## Beschreibung

Die Erfindung betrifft eine Vorrichtung zur Beaufschlagung flüssiger Medien mit gasförmigen Stoffen.

Die Überführung gasförmiger Stoffe in flüssige Medien spielt vor allem bei biologischen Systemen eine wichtige Rolle. Um Mikroorganismen wie z.B. Bakterien oder eukariotische Zellen mit gasförmigen Stoffen zu versorgen, müssen diese Stoffe zunächst in dem wässrigen Medium gelöst werden, in dem sich die Mikroorganismen befinden. Erst von hier aus gelangen diese Stoffe unmittelbar an die Mikrooganismen heran. Bei diesen Systemen ergibt sich jedoch die zusätzliche Schwierigkeit, daß mit den gasförmigen Stoffen häufig Fremdkeime und toxische Verbindungen übertragen werden, die unerwünscht sind.

Aus US-A-3 941 662 ist eine gattungsgemäße Vorrichtung bekannt, die zur Kultivierung tierischer Zellen dient. Hierbei findet die Kultivierung der Zellen in gasdurchlässigen Behältern statt, wobei die Gasversorgung von außen durch die Behälterwand erfolgt. Bei derartigen Vorrichtungen stellt sich für den zu überführenden Stoff ein Gleichgewicht zwischen der Gasphase und der Flüssigkeitsphase ein, das in der Regel nur einen unzureichenden Gasübertritt in die Flüssigkeitsphase ermöglicht. Infolgedessen wurde durch Erhöhung des Gasdruckes versucht, eine bessere Überführung des Gases in die Flüssigkeitsphase zu erreichen.

WO-A-8 912 385 beschreibt eine Vorrichtung zur Kultivierung von verschiedenen Zellen, bei der eine Gasversorgung ebenfalls über gasdurchlässige Membranen vorgenommen wird. Diese Vorrichtung wird dann einer bestimmten Gasatmosphäre ausgestzt, so daß das Gas durch die Membran zu den Zellen gelangen kann. Hierbei wird allerdings keine effektive Beaufschlagung des flüssigen Mediums erreicht, weil die Membranzellen nur seitlich miteinander verbunden in einer Ebene liegen und von dem Gas nicht geeignet angeströmt werden.

Eine weitere Vorrichtung zur Kultivierung von Zellen ist in US-A-3 184 395 beschrieben. Allendings läßt auch diese Druckschrift außer acht, daß zur effektiven Beaufschlagung eines flüssigen Mediums ein Gasstrom erforderlich ist, der für einen ständigen Diffusionsgradienten sorgt.

Weitere Vorrichtungen zur Kultivierung von Zellen sind in US-A-4 416 993 und WO-A-9 010 690 beschrieben. Auch mit diesen Vorrichtungen ist jedoch keine ausreichende Überführung von Gasen in eine flüssige Phase möglich.

Die Aufgabe der vorliegenden Erfindung ist es daher, eine Vorrichtung zur Beaufschlagung flüssiger Medien mit gasförmigen Stoffen bereitzustellen, mit der die geschilderten Nachteile beseitigt werden können. Insbesondere soll eine Vorrichtung geschaffen werden, mit der auch ohne besondere Druckerhöhung eine effektive Beaufschlagung möglicht ist.

Erfindungsgemäß erfolgt die Lösung der Aufgabe dadurch, daß eine Zahl von gasdurchlässigen und die flüssigen Medien enthaltenden Hohlkörpermenbranen im Abstand parallel zueinander und parallel zur Strömungsrichtung der gasförmigen Stoffe angeordnet ist.

Die erfindungsgemäße Vorrichtung hat den Vorteil, daß sie sich für verschiedene Anwendungen einsetzen läßt. Sie eignet sich zur Kultivierung von Mikroorganismen ebenso wie zur Luftreinigung. Dabei lassen sich sogar Gase in nur geringen Konzentrationen wirksam in eine Flüssigkeit überführen.

Weitere Ausgestaltungen der Erfindung werden durch die Unteransprüche beschrieben und nachfolgend anhand der in den Zeichnungen als bevorzugt dargestellten Ausführungsformen näher erläutert. Es zeigt:
- Fig. 1a: die erfindungsgemäße Vorrichtung in einer schematischen Seitenansicht im Schnitt A-A,
- Fig. 1b: die Vorrichtung in einem schematischen Querschnitt B-B,
- Fig. 2a: die Anordnung der einzelnen Hohlkörpermembranen in einer perspektivischen Seitenansicht,
- Fig. 2b: den Ausschnitt A in einer vergrößerten Darstellung,
- Fig. 3a: eine Hohlkörpermembran in einer schematischen Seitenansicht,
- Fig. 3b: eine Hohlkörpermembran im schematischen Längsschnitt C-C,
- Fig. 3c: eine Hohlkörpermembran im schematischen Querschnitt D-D,
- Fig. 4a und 4b: jeweils eine Hohlkörpermembran in einem schematischen Längsschnitt,
- Fig. 4c: eine Hohlkörpermembran in einem schematischen Querschnitt,
- Fig. 4d: den Ausschnitt B einer Hohlkörpermembran,
- Fig. 4e: den Ausschnitt C einer Hohlkörpermembran,
- Fig. 5: die Anordnung miteinander kombinierter Einzelvorrichtungen im Schnitt.

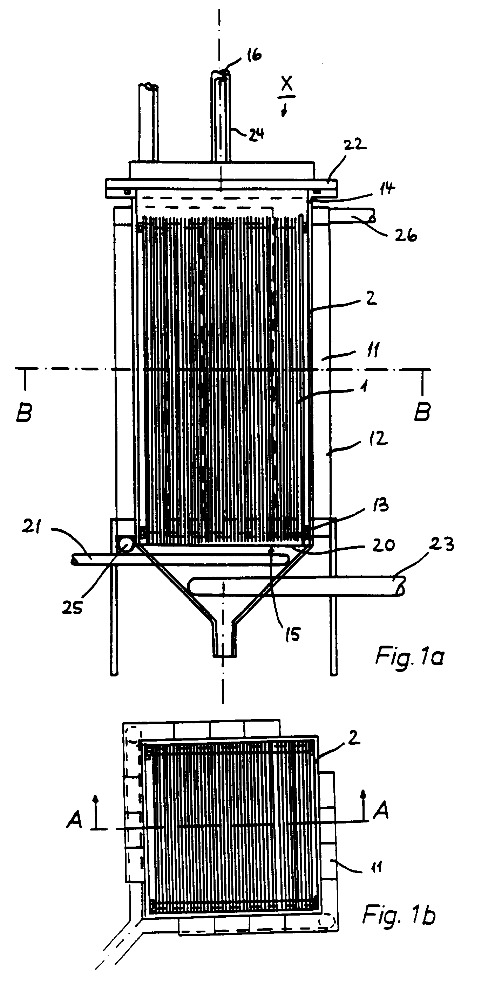

Die in Fig. 1 dargestellte erfindungsgemäße Vorrichtung X zeigt in einer bevorzugten Ausgestaltung einen reaktorähnlichen Aufbau mit einem geschlossenen Gehäuse 2, in dem eine Zahl von Hohlkörpermembranen 1 im Abstand zueinander angeordnet ist. Diese Hohlkörpermembranen 1 bilden zusammen ein Membranmodul, das mit dem Gehäuse 2 fest oder lösbar verbunden sein kann. Die einzelnen Hohlkörpermembranen 1 sind mit einem wässrigen Medium befüllt und werden von einem gasförmigen Medium angeströmt. Die Strömung verläuft dabei parallel zu den Außenflächen der Hohlkörpermembranen, um auf diese Weise eine Diffusion der Gase möglichst über die gesamte Membran verteilt zu erreichen. Am unteren Ende 13 des Gehäuses 2 kann ein Boden 20 ausgebildet sein, der einzelne Gaseinlaßöffnungen 15 aufweist. Durch die Gaseinlaßöffnungen 15 gelangt das Medium zu den Hohlkörpermembranen 1. Um einen möglichst geringen Strömungswiderstand zu erreichen, kann der Boden 20 auch fehlen. Je nach Anwendungsfall kann die Vorrichtung X unmittelbar in einen vorhandenen Gasstrom eingebracht werden. Andererseits ist es möglich, das zu überführende Medium über Transferleitungen 21 heranzuführen und in die Vorrichtung X einzuspeisen. Nachdem das gasförmige Medium die Hohlkörpermembranen 1 passiert hat, tritt es am oberen Ende 14 des Gehäuses 2 aus. In einer Abdeckung 22 sind hierzu einzelne Auslaßöffnungen vorgesehen. Es kann aber auch eine zentrale Gasauslaßöffnung 16 ausgebildet sein.

Je nach Zustand der zu überführenden Medien empfiehlt es sich, für die Hohlkörpermembranen 1 eine klimatisierte Umgebung zu erzeugen, um Austrocknungen der in den Hohlkörpermembranen 1 enthaltenen Flüssigkeiten zu verhindern. Die relative Luftfeuchtigkeit wird hierzu bevorzugt auf Werte zwischen 50% und 100% eingestellt, wobei eine definierte Klimazufuhr über Zuführleitungen 23 erfolgen kann und die Klimaabluft z.B. über eine zentral angeordnete Klimaabluftleitung 24 geführt wird. Durch eine im Mantel 11 angeordnete Temperiervorrichtung 12 kann die Temperatur der eingeströmten Medien bis zur Taupunktnähe abgesenkt werden. Auf diese Weise wird eine gleichbleibend hohe Luftfeuchtigkeit erzeugt. Die Temperiervorrichtung kann als kombinierter Heiz- und Kühlmantel ausgebildet sein und einen Solezulauf 25 und einen Soleablauf 26 aufweisen. Insgesamt kann so die Temperatur auf beliebige Werte eingestellt werden, je nach zu beaufschlagenden Medien. Unter Umständen kann auch ein Atmosphärenüberoder Unterdruck vorteilhaft sein, wenn das gasförmige Medium eine geringe oder hohe Löslichkeit besitzt.

Je nach Temperatur und relativer Luftfeuchtigkeit des eingeströmten, gasförmigen Mediums kann eine Mantelisolierung vorgenommen werden, durch die die Temperatur in der Vorrichtung X nahezu konstant und im angegebenen Bereich gehalten werden kann. Bei Temperaturen unterhalb von z.B. 0°C oder oberhalb von z.B. 50°C sollte die Mantelisolierung zusätzlich zur Temperiervorrichtung angebracht sein.

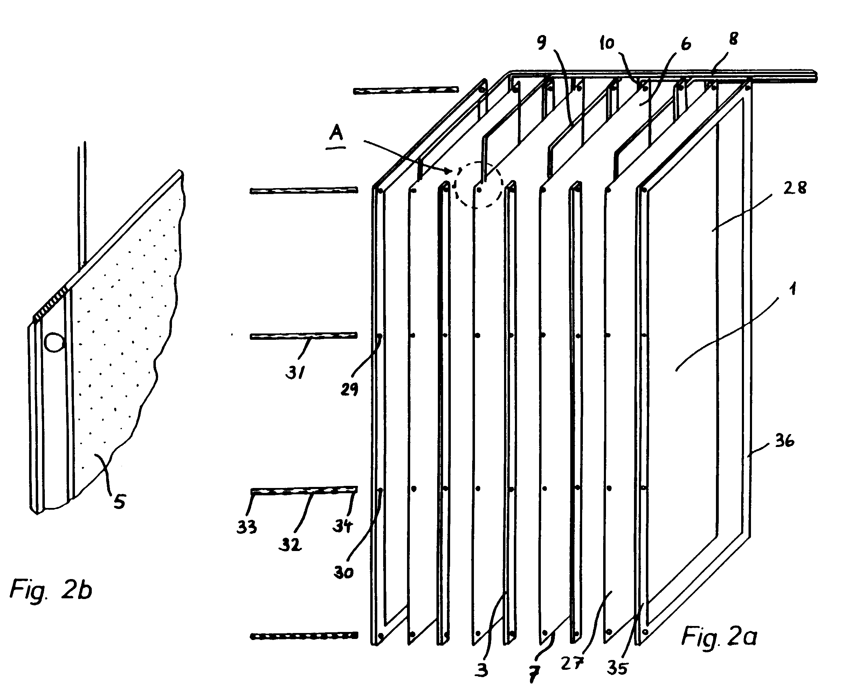

Die Anordnung der Hohlkörpermembranen 1 ist in Fig. 2a dargestellt. In einer besonderen Ausführungsform erkennt man einzelne Hohlkörpermembranen 1, die hier taschenförmig mit einem flächigen Zuschnitt ausgebildet und parallel zueinander angeordnet sind. Jeweils zwischen zwei Hohlkörpermembranen 1 sind Abstandselemente 3 vorgesehen. Diese können stabförmig ausgebildet sein und einen beliebigen, vorzugsweise rechteckigen Querschnitt aufweisen. Sie erstrecken sich entlang der seitlichen Endabschnitte 27, 28 der einzelnen Hohlkörpermembranen 1 und besitzen einen Querschnitt, so daß im Grunde ein beliebiger Abstand der einzelnen Hohlkörpermembranen 1 zueinander erreichbar ist. Je nach Strömungsgeschwindigkeit des gasförmigen Mediums sowie nach Art der verwendeten Hohlkörpermembranen 1 kann dieser etwa 2 mm betragen oder auch kleiner oder größer sein. Die Abstandselemente 3 stabilisieren gleichzeitig die Gesamtvorrichtung X. Sie können Bohrungen 29, 30 aufweisen, durch die Befestigungsbolzen 31, 32 geführt werden. Über Verschraubungen an den beiden Enden 33, 34 der Befestigungsbolzen 31, 32 können so die Hohlkörpermembranen zusammengehalten werden, wobei ein leicht handhabbares Modul entsteht. Zur weiteren Stabilisierung können insbesondere die äußeren Abstandselemente 35, 36 endabschnittsseitig miteinander verbunden sein. In einer nicht näher dargestellten Ausführungsform sind die Abstandselemente 3 am Gehäuse 2 angeformt und dienen auf diese Weise als Halterung für das Membranmodul.

Fig. 2b zeigt den Ausschnitt A, in dem einzelne Erhöhungen 5 auf der Oberfläche der Hohlkörpermembran zu erkennen sind. Diese Erhöhungen können durch Verstärkungen des Membranmaterials ausgebildet sein und sollen den Abstand zwischen zwei benachbarten Hohlkörpermembranen über die gesamte Seitenfläche gewährleisten.

Zur wiederholbaren Befüllung sind die Hohlkörpermembranen 1 an ihrem unteren Ende 7 verschlossen und weisen an ihrem oberen Ende 6 eine gemeinsame Versorgungseinrichtung 8 auf, wodurch sich die Hohlkörpermembranen 1 auch im eingebauten Zustand jederzeit befüllen lassen. Jede Hochlkörpermembran 1 ist hierzu mit einer Zuleitung 9 und einer Ableitung 10 verbunden. Über die Zuleitung 9 gelangt das wässrige Medium in die Hohlkörpermembranen 1 und kann schließlich über die AbLeitung 10 ausgetauscht werden.

Soll eine Kontamination durch unerwünschte Mikroorganismen verhindert werden, läßt sich das gesamte System sterilisieren. Die Hohlkörpermembranen 1 können auch z.B. mit Wasser vorgefüllt werden, das anschließend ausgetauscht wird. Die Füllung mit Wasser soll verhindern, daß die Seiten 37, 38 der Hohlkörpermembranen während der Sterilisation miteinander verkleben. Für den Fall, daß die Hohlkörpermembranen 1 nur zum einmaligen Gebrauch vorgesehen sind, können Zuführeinrichtungen fehlen.

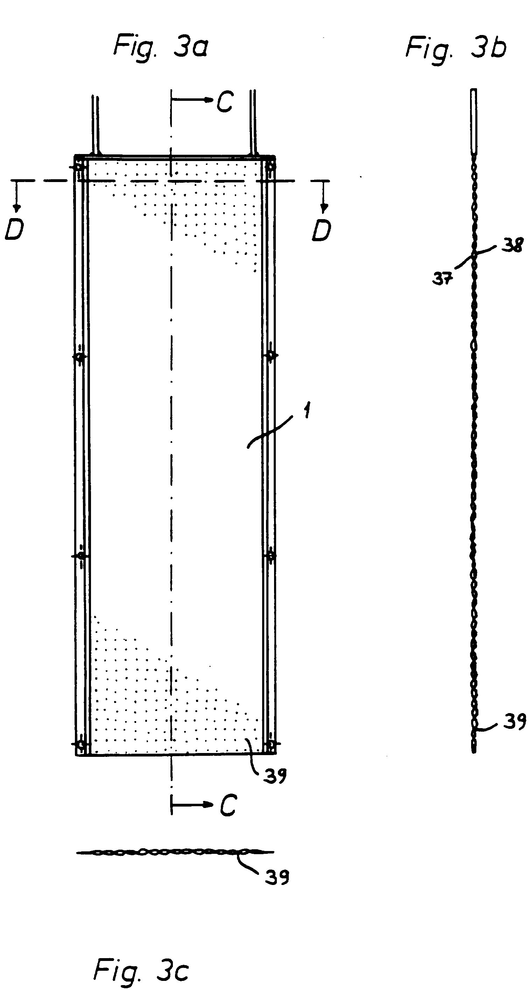

Eine einzelne Honlkörpermembran 1 ist in Fig. 3a bis 3c dargestellt. In einer bevorzugten Ausgestaltung weist diese Hohlkörpermembran 1 einen flächigen Zuschnitt auf. Die Hohlkörpermembran 1 ist allgemein taschenförmig ausgebildet und kann Verbindungen 39 zwischen den Seiten 37, 38 aufweisen, die z.B. über punktförmige Verschweißungen oder Verklebungen erhältlich sind und über die gesamte Hohlkörpermembran 1 verteilt sein können.

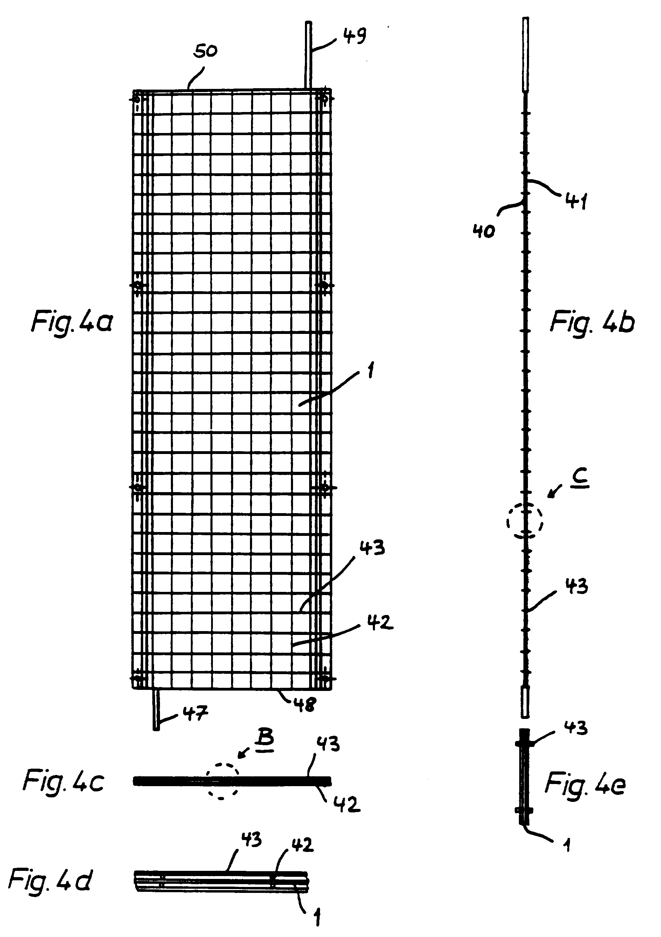

Eine Möglichkeit zur Versteifung der Hohlkörpermembran besteht darin, die Außenseiten 40, 41 mit dünnen Verfestigungselementen zu versehen, die z.B. als Längs- und Querfäden 42, 43 ausgebildet sind und aus einem beständigen Material wie z.B. Teflon^{R} bestehen. Solche Versteifungselemente können aber auch in die Membranseiten 37, 38 eingelassen sein, um eine möglichst glatte Oberfläche der Membran zu erzeugen. Eine entsprechende Ausgestaltung der Hohlkörpermembran 1 zeigt Fig. 4a bis 4e. In Abbildung 4a erkennt man ferner, daß Zu- und Ableitungen zur Befüllung der Hohlkörpermembran 1 auch an verschiedenen Seiten der Hohlkörpermembran 1 auch angeordnet sein können. Beispielsweise Kann eine Zuleitung 47 an der Unterseite 48 und eine Ableitung 49 an der Oberseite 50 vorgesehen sein. Durch diese Anordnung wird es möglich, den Inhalt einer Hohlkörpermembran 1 jederzeit nahezu vollständig auszutauschen.

Die Hohlkörpermembranen 1 bestehen aus einem gasdurchlässigen Material. Dieses kann in einem weiten Bereich temperaturbeständig sein und sich z.B. bei 121°C sterilisieren lassen. Das Material sollte mechanischen Belastungen widerstehen und weitgehend resistent gegenüber chemischen Verbindungen sein. Als besonders vorteilhaft hat sich hierbei eine ca. 25 .m starke Polyurethanfolie erwiesen, die von verschiedenen Herstellern z.B. unter dem Handelsnamen Walopur (Wolff Walsrode AG) erhältlich ist. Diese kann längs- und/oder quergespritzt sein. Darüber hinaus sind aber auch andere gasdurchlässige Materialien wie z.B. Silikongummi verwendbar.

Die Hohlkörpermembranen 1 können auch schlauch- oder röhrenförmig ausgebildet sein, wodurch ein Membranmodul aus einem Bündel von Hohlkörpermembranen gebildet wird, das den Vorteil einer flexiblen Anordnung hat und z.B. in gebogenen oder abgewinkelten Luftschächten eingesetzt werden kann.

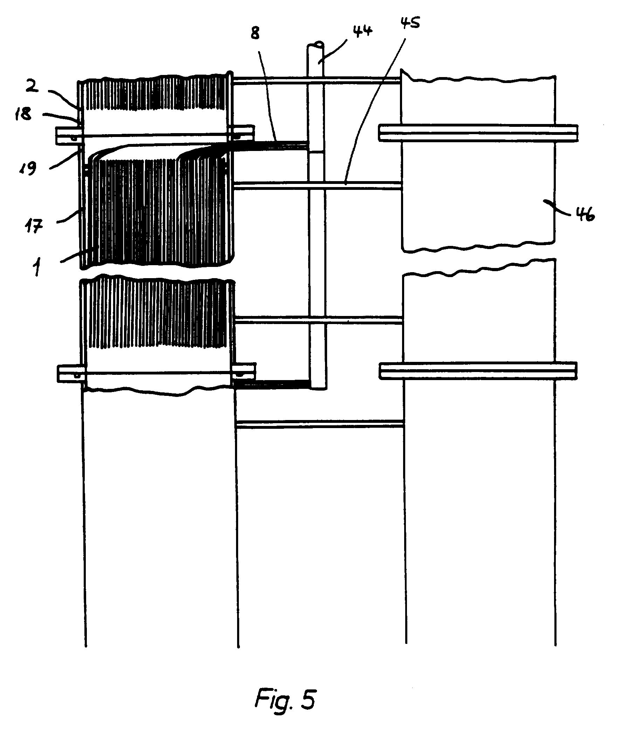

Für den Fall, daß größere Mengen gasförmiger Stoffe überführt werden sollen, muß eine entsprechend große Oberfläche der Hohlkörpermembranen 1 vorliegen. Dies kann z.B. dadurch erreicht werden, daß eine Vielzahl von einzelnen Hohlkörpermembranen nebeneinander angeordnet werden. Andererseits lassen sich aber auch mehrere Vorrichtungen X miteinander kombinieren, wie dies in Fig. 5 dargestellt ist, um auf diese Weise den Stoffaustausch insgesamt zu erhöhen. Hierzu wird das untere Ende 18 eines Gehäuses 2 mit dem oberen Ende 19 eines anderen Gehäuses 17 verbunden, so daß ein kontinuierlicher Gasstrom durch nacheinander angeordnete Membranmodule geleitet werden kann. Zwischen nebeneinander angeordneten Gehäusen 17, 46 können zusätzlich Verbindungselemente 45 ausgebildet sein. Bei einer solchen Kombination einzelner Vorrichtungen können auch die Versorgungseinrichtungen 8 für einzelne Membranmodule untereinander verbunden sein. Hierzu wird eine gemeinsame Leitung 44 vorgesehen, über die sämtliche Hohlkörpermembranen 1 gemeinsam befüllbar und auch wieder entleerbar sind.

Durch die Kombinierbarkeit einzelner Vorrichtungen X wird es vor allem möglich, auch größere Mengen gasförmiger Stoffe zu überführen. Können beim einmaligen Durchströmen einzelner oder miteinander kombinierter Vorrichtungen X die gasförmigen Stoffe nicht quantitativ entfernt werden, ist eine Rezirkulation ebenfalls durchführbar.

### Verwendung der erfindungsgemäßen Vorrichtung zur Beaufschlagung wässriger Suspensionen von Mikroorganismen

Mit der erfindungsgemäßen Vorrichtung lassen sich Mikroorganismen gezielt untersuchen. Durch die Beaufschlagung von Rein- oder Mischkulturen können beispielsweise Erkenntnisse über die Verwertbarkeit verschiedener Gase, vor allem aber auch über Toxizitäten einzelner Stoffe gewonnen werden. Die Diffusion der Gase über die große Membranoberfläche führt hierbei zu einer schonenden Beaufschlagung der Mikroorganismen, was insbesondere bei der Behandlung pflanzlicher und tierischer Zellkulturen von großer Bedeutung ist.

Die erfindungsgemäße Vorrichtung eignet sich vor allem zur Beaufschlagung wässriger Suspensionen von Mikroorganismen mit solchen Stoffen, die von dem in der Suspension enthaltenden Mikroorganismen umwandelbar sind. Auf diese Weise lassen sich z.B. gasförmige Schwefelverbindungen wie z.B. Schwefelwasserstoff aber auch Kohlenmonoxid, Kohlendioxid, gasförmige Kohlenwasserstoffe oder/und Stickoxide aus einem Gasstrom beliebiger Art entfernen und umsetzen.

Gasförmige Schwefelverbindungen wie Schwefelwasserstoff lassen sich beispielsweise durch den Einsatz von Thiobacillen beseitigen, während eine Reihe anderer Bakterienarten z.B. der Gattung Pseudomonas, Alcaligenes, Nocardia und Methylomonas für die Oxidation von Kohlenmonoxid bzw. von Kohlenwasserstoffen einsetzbar sind.

Zur Beseitigung von Stickoxiden können die Hohlkörpermembranen 1 mit nitrifizierenden Bakterien z.B. der Gattung Nitrobacter befüllt werden. Diese Organismen oxidieren Stickstoffmonoxid, ohne daß zusätzliche Substrate eingesetzt werden müssen. Beispielsweise lassen sich die nitrifizierenden Bakterien in einer wässrigen Lösung mit folgender Zusammensetzung verwenden:

**Tabelle 1 :**

| Nährlösung | |
|---|---|
| NaCl | 0,5 g/l |
| MgSO₄ ^{˙} 7 H₂O | 0,05 g/l |
| KH₂PO₄ | 0,15 g/l |
| FeSO₄ ^{˙} 7 H₂O | 0,15 g/l |
| (NH₄)₆Mo₇O₂₄ ^{˙} 4 H₂O | 0,05 g/l |
| CaCO₃ | 0,01-1,0 g/l |
| pH 7,4 - 8,0 | |

Dieser wässrigen Lösung werden die Bakterien in möglichst hoher Zellkonzentration zugesetzt, wobei 10⁷ Zellen/ml mindestens vorliegen sollten. Bei geringer Zellkonzentration verlangsamt sich der Stoffumsatz.

Die gleiche Lösung eignet sich auch zur Anzucht von Methylomonas, durch dessen Verwendung gasförmige Kohlenwasserstoffe, wie z.B. Methan, beseitigt werden können. Häufig ist zu beobachten, daß durch die Verwendung nur einer bestimmten Bakterienart verschiedene gasförmige Stoffe eliminiert werden können.

Die Beseitigung von Stickstoffmonoxid läßt sich aber auch durch den Einsatz von methylotrophen Bakterien (z.B. der Gattung Methylobacterium) erreichen.

### Beispiel 1 (einmalige Beaufschlagung)

Als Versuchsanlage weist die erfindungsgemäße Vorrichtung eine mögliche Anzahl von 29 Hohlkörpermembranen auf, die taschenförmig ausgebildet sind. Jede Membran enthält 25 ml einer Bakteriensuspension mit 5 x 10⁸ Zeilen/ml mit einem Naturisolat der Gattung Methylobacterium. Als Substrat-Additiv dient Methanol, das in einer 1%igen Konzentration der mineralischen Grundlösung gemäß der in Tabelle 1 beschriebenen Zusammensetzung zugegeben wurde. Die Vorrichtung wurde anschließend mit einem NO-Luft-Gemisch mit einer NO-Konzentration von 4,8 bis 5,1 Volumenanteile pro Million (vpm) beaufschlagt und verschlossen. Nach einer Stunde war in der Vorrichtung eine Abnahme der NO-Konzentration von 98% erreicht.

### Beispiel 2 (kontinuierliche Beaufschlagung)

Weiterhin wurde die mikrobielle NO-Abbaurate ermittelt, die sich im Durchflußbetrieb bei 29 Hohlkörpermembranen wie in Beispiel 1 einstellt. Vor Eintritt in die Vorrichtung und nach Austritt wurde die NO-Konzentration des Gases gemessen. Die NO-Konzentration des verwendeten NO-Luft-Gemisches betrug wiederum 4,8 bis 5,1 vpm. Bei einer Durchflußmenge von 12 l/h wurde die anfängliche NO-Konzentration um 40% reduziert. Bei einer Verdoppelung der Durchflußmenge auf 24 l/h wurden 33% des NO-Gehaltes aus dem Gas eliminiert, während bei einer Durchflußmenge von 48 l/h sogar eine NO-Abnahme von 25% ereichbar war.

Methanoloxidierende Bakterien der Gattungen Ancylobacter und Rhenobacter wurden ebenfalls untersucht. Die Beaufschlagung dieser Organismen erfolgte wie bei den methylotrophen Bakterien in einem mineralischen Medium ohne Stickstoffquelle mit 1% Methanol als Substrat. Es konnte gezeigt werden, daß der gemessene NO-Verbrauch bei Anwendung der erfindungsgemäßen Vorrichtung direkt abhängig ist von der eingesetzten Bakterienmenge. Vertreter der Gattung Rhenobacter haben eine höhere spezifische Aktivität als die von Ancylobacter. Bei 5°C ist der NO-Verbrauch wesentlich geringer als bei 28°C.

### Beispiel 3 (Temperaturabhängigkeit)

In einer Suspension fakultativ methylotropher Bakterien der Gattung Methylobacterium wurde die NO-Abnahme in Abhängigkeit von der Temperatur untersucht. Es wurde eine Versuchsanordnung wie in Beispiel 2 gewählt. Die Durchflußmenge NO-haltiger Luft betrug 48 l/h. Aus Tabelle 2 geht hervor, daß die maximale mikrobielle NO-Abbaurate zwischen 10 und 15°C liegt. Bei 30°C ist der NO-Abbau niedriger als bei 5°C.

**Tabelle 2 :**

| NO-Abnahme in Abhängigkeit von der Temperatur | |
|---|---|
| Temperatur | NO-Abnahme |
| 5°C | 20 % |
| 10°C | 35 % |
| 15°C | 32,5 % |
| 20°C | 32 % |
| 25°C | 25 % |
| 30°C | 12 % |

Da außer bei nitrifizierenden Bakterien weder Nitrit noch Nitrat gefunden wurde, wird eine oxidative Umwandlung des eingesetzen Stickstoffmonoxids ausgeschlossen. Eine reduktive Umwandlung von NO zu N₂O und N₂ ist als Denitrifikation bekannt. In der Natur läuft dieser Prozeß allerdings unter anaeroben und nicht wie bei den durchgeführten Versuchen unter aeroben Bedingungen ab. Eine aerobe biologische Umwandlung von NO zu N₂O und N₂ ist neu. Sie gleicht nicht der klassischen Denitrifikation, da ein Zellwachstum -anders als bei der Denitrifikation- bei den durchgeführten Versuchen nicht nachweisbar war.

Seit kurzem ist bekannt, daß NO ein wichtiger Effektor in der Regulation des Zellstoffwechsels ist. NO bindet kovalent an Eisen-Schwefelproteine und bildet eine Dinitroverbindung. In methylotrophen Bakterien wirkt NO zusammen mit Eisen als ein zusätzlicher Effektor auf die Aktivität der Adenylatcyclase. Adenylatcyclase synthetisiert cAMP, das seinerseits die Synthese für induzierbare Enzyme reguliert. Ist viel cAMP in der Zelle vorhanden, so wird die Transkription von induzierbaren Enzymen ermöglicht. Durch NO wird die Adenylatcyclase aus methylotrophen Bakterien und Renobacter vacuolatum aktiviert, die von Guamilatcyclase jedoch gehemmt. Demnach ist NO ein positiver Effektor bei der Enzyminduktion verschiedener Bakterien.

Es ist anzunenmen, daß diejenigen Bakterien, bei denen NO ein Effektor in der Regulation des Zellstoffwechsels ist, auch die Konzentration von NO ihrerseits regulieren. Werden solche Backterien in die Membranen der erfindungsgemäßen Vorrichtung eingebracht, kann durch die Stoffwechselleistung dieser Organismen eine Beseitigung von NO erreicht werden.

In einem weiteren Versuch wurde der NO-Verbrauch noch dadurch gesteigert, daß einer Zellsuspension von Methylobacterium Elektronenüberträger zugesetzt wurden. So konnte der NO-Verbrauch Methanol oxidierender Bakterien durch Fe³- und PQQ-Gaben gesteigert werden (PQQ=Metoxatin=2,7,9-Tricarboxy-1H-pyrrolo (2, 3-f) - chinolin-4,5-dion. Die Methanol-Dehydrogenase methylotropher Bakterien ist nämlich nicht mit der Reduktion von NAD⁺ sondern mit der Elektronenübertragung auf Metoxatin gekoppelt. Das Chinon hat ein Redoxpotential von E'ₒ = +120 mV. NO wird somit an Fe³⁺ gebunden und mit reduziertem PQQ zu N₂O reduziert. Die Zellen methylotropher Bakterien liefern hierbei die Eiektronen aus der Methanoloxidation.

### Beispiel 4 (Kombination von Bakterien mit Elektronenüberträgern)

In Vorversuchen wurden 25 ml einer Zellsuspension (3,2 x 10⁸ Zellen/ml) von Methylobacterium spec. in einer 1,3 1-schraubdeckelflasche in Gegenwart von 1% Methanol und 4,85 vpm NO bei 28°C geschüttelt. Es wurde der NO-Verbrauch nach 6 h gemessen.

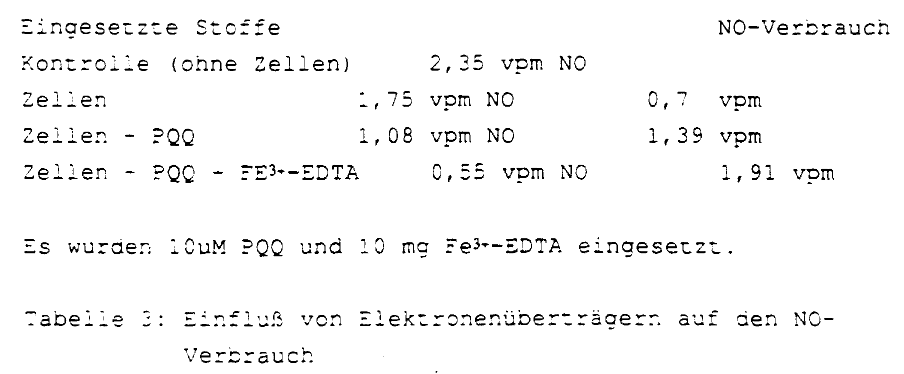

Die Ergebnisse aus diesem Vorversuch lassen sich auf die erfindungsgemäße Vorrichtung übertragen. Durch Beaufschlagung wässriger Suspensionen geeigneter Mikroorganismen sind somit in Gegenwart von Elektronenüberträgern gesteigerte NO-Umsatzraten zu erzielen.

### Verwendung der erfindungsgemäßen Vorrichtung zur Beaufschlagung von zellfreien Lösungen

Aufgrund der gewonnen Erkenntnisse bei der Beaufschlagung suspendierter Mikroorganismen in Gegenwart von Elektronenüberträgern wurde in weiteren Versuchen die Beaufschlagung zellfreier Lösungen zur Beseitigung von Stickoxiden untersucht. Es konnte gezeigt werden, daß ein NO-Verbrauch auch bei Beaufschlagung einer wässrigen Lösung erreichbar ist, die keine Mikroorganismen enthält. Durch verschiedene Reduktionsmittel konnte eine Beseitigung von NO erreicht werden. So wurde z.B. NO an Fe³⁺ gebunden und durch einen Elektronenüberträger reduziert.

### Beispiel 5 (NO-Reduktion mittels Elektronenüberträgern)

In weitere Vorversuchen wurden 25 ml einer mineralischen Lösung (pH 7,6) ohne Bakterien mit 1 mM Ascorbinsäure in einer 1,3 l-Schraubdeckelflasche mit und ohne Zusätze in Gegenwart von 4,26 vpm NO bei 28°C geschüttelt. Als Zusätze wurden 10 µM PQQ und 10 µM Fe³⁺-EDTA/l verwendet. Tabelle 4 zeigt die Ergebnisse dieser Versuche.

**Tabelle 4 :**

| Zellfreie NO-Reduktion | | | | |
|---|---|---|---|---|
| Ansatz | NOₜ₀ | NOₜ₌₆ₕ | Δ NO | Δ NO - Kontrolle |
| Ascorbinsäure | 4,26 | 3,89 | 0,37 | - |
| Ascorbinsäure + PQQ | 4,26 | 2,40 | 1,86 | 1,49 |
| Ascorbinsäure + PQQ + Fe³⁺-EDTA | 4,57 | 1,47 | 3,1 | 2,73 |

### Beispiel 6

Unter Verwendung jeweils einer Hohlkörpermembran ist schließlich der Versuch gemäß Beispiel 5 wiederholt worden und zwar in der Weise, daß jeweils eine taschenförmig ausgebildete Hohlkörpermembran in eine gasdichte Laborflasche mit einem Volumen von 1,3 l gehängt wurde. In Vergleichsversuchen wurde diese Membran gefüllt mit
- 1.: Nährlösung pH 7,6 (Kontrolle)
- 2.: Nährlösung mit Ascorbinsäure (1mM) und PQQ (10µM)
- 3.: Nährlösung mit Ascorbinsäure (1mM), PQQ (10µM) und Fe₃ ⁺-EDTA (10 mg/l)
- 4.: Nährlösung mit Ascorbinsäure (1mM) und Fe₃+-EDTA (10 mg/l)

Die jeweiligen Flaschen wurden mit einen NO/N₂-Preßluft-Gemisch gefüllt und anschließend gasdicht im Dunkeln in einen 28°C Raum gestellt. Nach 6 Stunden wurde die NO-Konzentration in den verschiedenen Flaschen bestimmt. Die Ergebnisse sind in der folgenden Tabelle dargestellt:

**Tabelle 5 :**

| Zellfreie NO-Reduktion mittels Membranen (Angaben in vpm) | | | | |
|---|---|---|---|---|
| Ansatz | NOₜ₀ | NOₜ₌₆ₕ | Δ NO | Δ NO - Kontrolle |
| Kontrolle | 4,54 | 2,50 | 2,04 | - |
| Ascorbins.+PQQ | 4,88 | 2,01 | 2,87 | 0,83 |
| Ascorbins.+PQQ+Fe | 4,91 | 2,07 | 2,84 | 0,80 |
| Ascorbins.+Fe | 4,61 | 1,78 | 2,83 | 0,79 |

Nach allem kann somit die erfindungsgemäße Vorrichtung insbesondere zur Luftreinigung verwendet werden. Durch die Verwendung dieser Vorrichtung können z.B. Rauch- und Abgase aus Feuerungsanlagen oder Straßentunnels gereinigt werden.

## Patentansprüche

1. Vorrichtung zur Beaufschlagung flüssiger Medien mit gasförmigen Stoffen, dadurch gekennzeichnet, daß eine Zahl von gasdurchlässigen und die flüssigen Medien enthaltenden Hohlkörpermembranen 1 im Abstand parallel zueinander und parallel zur Strömungsrichtung der gasförmigen Stoffe angeordnet ist.

2. Vorrichtung nach Anspruch 1, dadurch gekennzeichnet, daß die Hohlkörpermembranen 1 aus einer gasdurchlässigen Folie ausgebildet sind.

3. Vorrichtung nach Anspruch 1 oder 2, dadurch gekennzeichnet, daß die Hohlkörpermembranen 1 in sich geschlossen sind oder an ihrem unteren Ende 7 verschlossen sind und an ihrem oberen Ende 6 über eine gemeinsame Versorgungseinrichtung 8 miteinander verbunden sind.

4. Vorrichtung nach einem der Ansprüche 1 bis 3, dadurch gekennzeichnet, daß die Hohlkörpermembranen 1 jeweils eine Zu- und eine Ableitung 9, 10 für die flüssigen Medien aufweisen.

5. Vorrichtung nach einem der Ansprüche 1 bis 4, dadurch gekennzeichnet, daß die Hohlkörpermembranen 1 als Modul ausgebildet und in einem Gehäuse 2 entnehmbar angeordnet sind.

6. Vorrichtung nach einem der Ansprüche 1 bis 5, dadurch gekennzeichnet, daß das Gehäuse 2 einen Mantel 11 mit einer Temperiereinrichtung 12 aufweist.

7. Vorrichtung nach einem der Ansprüche 1 bis 6, dadurch gekennzeichnet, daß das Gehäuse 2 am unteren Ende 13 Gaseinlaßöffnungen 15 und am oberen Ende 14 Gasauslaßöffnungen 16 aufweist.

8. Vorrichtung nach einem der Ansprüche 1 bis 7, dadurch gekennzeichnet, daß mehrere Gehäuse 2, 17 miteinander kombinierbar sind, wobei das untere Ende 18 eines Gehäuses 2 mit dem oberen Ende 19 eines anderen Gehäuses 17 verbindbar ist und hierdurch einzelne Membranmodule nacheinander angeordnet sind.

9. Verwendung der Vorrichtung nach einem der Ansprüche 1 bis 8 zur Luftreinigung, insbesondere zur Reinigung von Rauch- und Abgasen aus Feuerungsanlagen oder Straßentunnels.

10. Vorrichtung zur Entfernung von Stickoxiden, gasförmigen Schwefelverbindungen, gasförmigen Kohlenwasserstoffen und Kohlenmonoxid, dadurch gekennzeichnet, daß eine Zahl gasdurchlässiger Hohlkörpermembranen 1 im Abstand parallel zueinander und parallel zur Strömungsrichtunq der zu entfernenden Stoffe angeordnet ist, wobei die Hohlkörpermembranen 1 eine wässrige Suspension von Mikroorganismen zur Umsetzung der Stoffe bzw. zellfreie Lösungen zur Umsetzung von Stickstoffmonoxid enthalten.

11. Vorrichtung nach Anspruch 10, dadurch gekennzeichnet, daß die Hohlkörpermembranen 1 eine wässrige Suspension von Mikroorganismen der Gattung *Pseudomonas*, *Alkaligenes, Nocardia,* *Methylomonas* oder von methylotrophen oder nitrifizierenden Bakterien enthalten.

## Claims

1. Device for feeding gaseous substances into liquid media, characterized in that a number of hollow membrane bodies 1 which are permeable to gas and contain the liquid media are arranged with a distance between them and parallel to the direction of flow of the gaseous substances.

2. Device according to claim 1 , characterized in that the hollow membrane bodies 1 are made of gas-permeable film.

3. Device according to claim 1 or 2, characterized in that the hollow membrane bodies 1 are completely closed or closed at the lower end 7 and share a common supply facility 8 at the upper end 6.

4. Device according to one of claims 1 to 3, characterized in that the hollow membrane bodies 1 exhibit both one feed and one outlet line 9, 10 for the liquid media.

5. Device according to one of claims 1 to 4, characterized in that the hollow membrane bodies 1 are developed as a module and arranged in a housing 2 so that they can be removed.

6. Device according to one of claims 1 to 5, characterized in that the housing 2 includes a jacket 11 with a temperature control system 12.

7. Device according to one of claims 1 to 6, characterized in that the housing 2 includes a lower end 13 with gas inlet openings 15 and an upper end 14 with gas outlet openings 16.

8. Device according to one of claims 1 to 7, characterized in that several housings 2, 17 are combined with one another whereby the lower end 18 of one housing 2 can be joined with the upper end 19 of another housing 17, and single membrane modules are arranged one after the other.

9. Use of the device according to any one of claims 1 to 8 for the purification of air, especially the purification of flue gas and exhaust of combustion plants or road tunnels.

10. Device for removing nitrogen oxides, gaseous sulfur compounds, gaseous hydrocarbons and carbon monoxide, characterized in that a number of hollow membrane bodies 1 which are permeable to gas are arranged with a distance between them and parallel to the direction of flow of the gaseous substances, whereby the hollow membrane bodies 1 contain a watery suspension of microorganisms to convert the substances or cell-free solutions to convert nitrogen monoxide respectively.

11. Device according to claim 10, characterized in that the hollow membrane bodies 1 contain a watery suspension of microorganisms of the genera *Pseudomonas, Alkaligenes,* *Nocardia, Methylomonas* or of methylotrophic or nitrifying bacteria.

## Revendications

1. Dispositif pour l'admission de milieux liquides contenant des substances gazeuses, caractérisé en ce que plusieurs membranes 1 de corps creux perméables au gaz et contenant les milieux liquides sont disposées à distance parallèlement entre elles et parallèlement au sens d'écoulement des substances gazeuses.

2. Dispositif selon la revendication 1, caractérisé en ce que les membranes de corps creux 1 sont constituées par une pellicule perméable au gaz.

3. Dispositif selon la revendication 1 ou 2, caractérisé en ce que les membranes de corps creux 1 sont fermées sur elles-mêmes ou sont fermées à leur extrémité inférieure 7 et sont reliées entre elles, à leur extrémité supérieure 6, par l'intermédiaire d'un dispositif commun d'alimentation 8.

4. Dispositif selon l'une des revendications 1 à 3, caractérisé en ce que les membranes de corps creux 1 présentent chacune une conduite d'amenée et d'évacuation 9, 10 pour les milieux liquides.

5. Dispositif selon l'une des revendications 1 à 4, caractérisé en ce que les membranes de corps creux 1 sont conçues comme un module et sont disposées de manière amovible dans un boîtier 2.

6. Dispositif selon l'une des revendications 1 à 5, caractérisé en ce que le boîtier 2 présente une enveloppe 11 munie d'un dispositif 12 d'équilibrage de la température.

7. Dispositif selon l'une des revendications 1 à 6, caractérisé en ce que le boîtier 2 présente, à l'extrémité inférieure 13 des ouverture d'entrée de gaz 15 et, à l'extrémité supérieure 14 des orifices de sortie de gaz 16.

8. Dispositif selon l'une des revendications 1 à 7, caractérisé en ce que plusieurs boîtiers 2, 17 sont combinables entre eux, l'extrémité inférieure 18 d'un boîtier 2 pouvant être reliée à l'extrémité supérieure 19 d'un autre boîtier 17, ce qui permet de disposer chacun des modules de membranes les uns derrière les autres.

9. Utilisation du dispositif selon l'une des revendications 1 à 8 pour la purification de l'air, en particulier pour la purification de gaz de fumée et d'échappement provenant d'installations de combustion ou de tunnels routiers.

10. Dispositif pour l'élimination d'oxyde d'azote, de liaisons gazeuses de soufre, d'hydrocarbures gazeux et de monoxydes de carbone, caractérisé en ce que plusieurs membranes de corps creux 1 perméables au gaz sont disposées parallèlement entre elles et parallèlement au sens d'écoulement des substan ces à éliminer, les membranes de corps creux 1 contenant une suspension aqueuse de micro-organismes pour la décomposition des substances, respectivement des solutions sans cellulose pour la décomposition du monoxyde d'azote.

11. Dispositif selon la revendication 10, caractérisé en ce que les membranes de corps creux 1 contiennent une suspension aqueuse de micro-organismes du genre Pseudomonas, Alkaligènes, Nocardia, Methylomonas, ou de bactéries méthylotrofènes ou nitrifiantes.
